# EUROPEAN PATENT APPLICATION

(11) **EP 4 570 177 A1**
(43) Date of publication of application: **18.06.2025**
(21) Application number: 24218960.3
(22) Date of filing: 11.12.2024
(51) Int. Cl.: A61B 5/1473

(54) **INVASIVE MULTI-ELECTRODE ELECTROCHEMICAL SENSOR**

(30) Priority: 12.12.2023 TW 112148320
(71) Applicant: UltraE Co. Ltd., Taichung City 412 (TW)
(72) Inventor: Chung, Hsieh-Hsun, 412 Taichung City (TW); Chang, Jen-Lin, 412 Taichung City (TW)
(74) Representative: Straus, Alexander

(57) **Abstract**

An invasive multi-electrode electrochemical sensor includes a substrate, plural wire electrodes and plural gold fingers. Each wire electrode includes an electrically conductive core and an insulating sheath that substantially covers, but exposes a proximal end and a distal end of, the electrically conductive core. Each wire electrode has a substrate section provided on the substrate and an invasion section extending outward from an edge of the substrate. The proximal end and distal end of the electrically conductive core of each wire electrode are located at the substrate section and invasion section of the wire electrode respectively. The invasion sections are at least partially wound around one another spirally. The electrically conductive core in the substrate section of each wire electrode is electrically connected to the corresponding gold finger. At least a part of the gold fingers is printed on the substrat by screen printing.

## Description

### BACKGROUND OF THE INVENTION

### 1. Technical Field

The present invention relates to an invasive electrochemical sensor.

### 2. Description of Related Art

Existing electrochemical sensors can be used in fluid detection and in that case are typically structured as test strips. An electrochemical test strip generally has a detection area to which a to-be-tested solution can be transferred in the form of a liquid drop or that can be immersed in the to-be-tested solution. However, existing electrochemical test strips cannot be used to perform invasive detection. When it comes to the continuous monitoring of biological/physiological parameters, an invasive detector is often more suitable than an existing non-invasive electrochemical test strip.

Therefore, it is an issue worth consideration by those working in the field to which the present invention pertains to provide an invasive electrochemical sensor that also has a relatively low production cost.

### BRIEF SUMMARY OF THE INVENTION

The primary objective of the present invention is to provide a relatively low-cost invasive electrochemical sensor.

To achieve the above and other objectives, the present invention provides an invasive multi-electrode electrochemical sensor (hereinafter also referred to as the electrochemical sensor for short) that includes a substrate, a plurality of wire electrodes and a plurality of gold fingers provided on the substrate. Each wire electrode includes an electrically conductive core and an insulating sheath. Each insulating sheath substantially covers the corresponding electrically conductive core while leaving a proximal end and a distal end of the corresponding electrically conductive core exposed. Each wire electrode has a substrate section and an invasion section. The proximal end and the distal end of the electrically conductive core of each wire electrode are located at the substrate section and the invasion section of the wire electrode respectively. The substrate section of each wire electrode is located on the substrate. The invasion section of each wire electrode extends outward from an edge of the substrate, and the invasion sections of the wire electrodes are at least partially wound around one another in a spiral manner. The electrically conductive core in the substrate section of each wire electrode is electrically connected to the corresponding gold finger. At least a part of the gold fingers is printed on the substrat by screen printing.

The present invention provides an invasive electrochemical sensor by using multiple spirally wound wire electrodes. Screen-printed gold fingers are used to electrically connect to the wire electrodes respectively. It has many advantages such as low cost, disposable, small size and sensitive reactivity, thereby solving the shortcomings of the existing technology.

### BRIEF DESCRIPTION OF THE SEVERAL VIEWS OF THE DRAWINGS

FIG. 1 is a perspective view of the first embodiment of the present invention.
FIG. 2 is an exploded view of the first embodiment of the present invention.
FIG. 3 is another exploded view of the first embodiment of the present invention, showing in particular how each electrically conductive core is electrically connected to the corresponding gold finger by soldering.
FIG. 4 is a partial enlarged view of the first embodiment of the present invention.
FIG. 5 shows sectional views of the distal ends of four electrically conductive cores.
FIG. 6 shows a sectional view of the distal end of an electrically conductive core in another embodiment.
FIG. 7 shows the electrochemical sensor of the present invention used in combination with an electrochemical sensing relay.
FIG. 8 shows a way of implementing the invasion sections of the wire electrodes in the present invention.
FIG. 9 shows another way of implementing the invasion sections of the wire electrodes in the present invention.
FIG. 10 shows the reproducibility test results of the electrochemical sensor of the present invention.
FIG. 11 shows cyclic voltammogram traces obtained by immersing the electrochemical sensor of the present invention in aqueous hydrogen peroxide solutions of different concentrations.
FIG. 12 shows a current-time graph obtained by detecting an aqueous hydrogen peroxide solution with the electrochemical sensor of the present invention by amperometry.
FIG. 13 shows a linear regression line fitted to the data points on a current-concentration graph obtained by detecting an aqueous hydrogen peroxide solution with the electrochemical sensor of the present invention by amperometry.
FIG. 14 shows still another way of implementing the invasion sections of the wire electrodes in the present invention.
FIG. 15 shows yet another way of implementing the invasion sections of the wire electrodes in the present invention.
FIG. 16 shows another way of implementing the electrochemical sensor of the present invention.

### DETAILED DESCRIPTION OF THE INVENTION

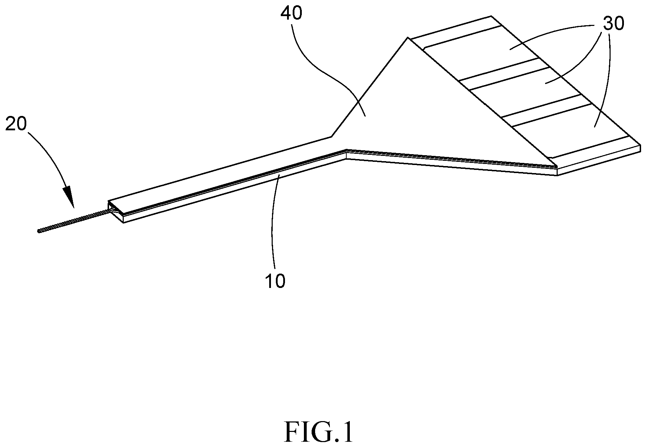

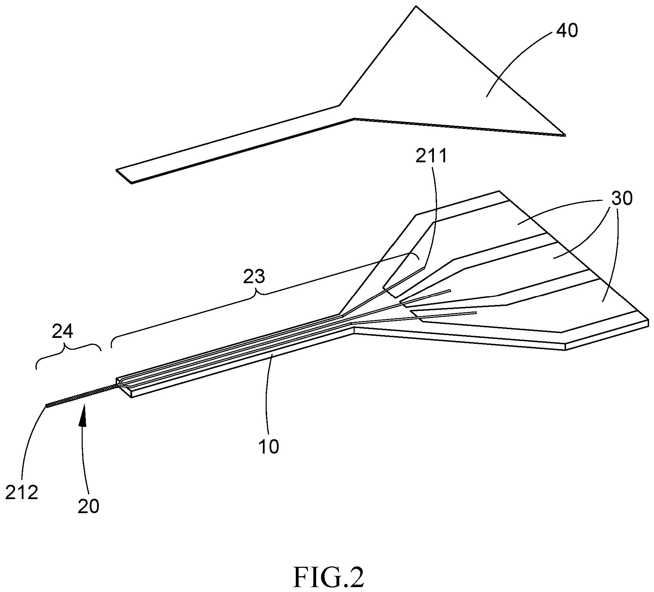

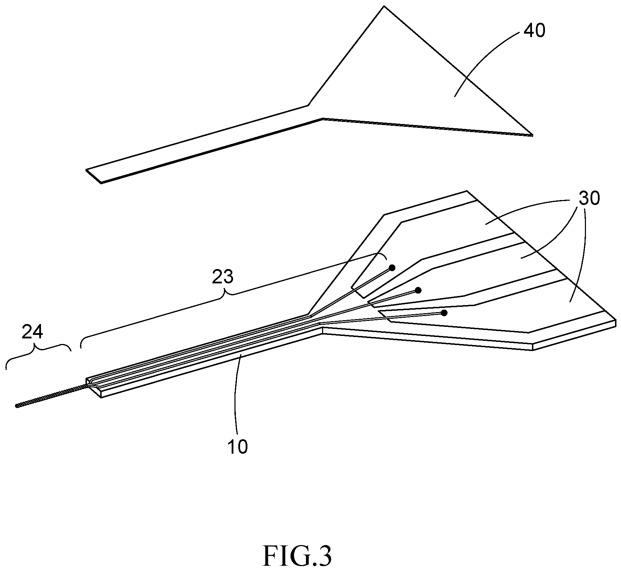

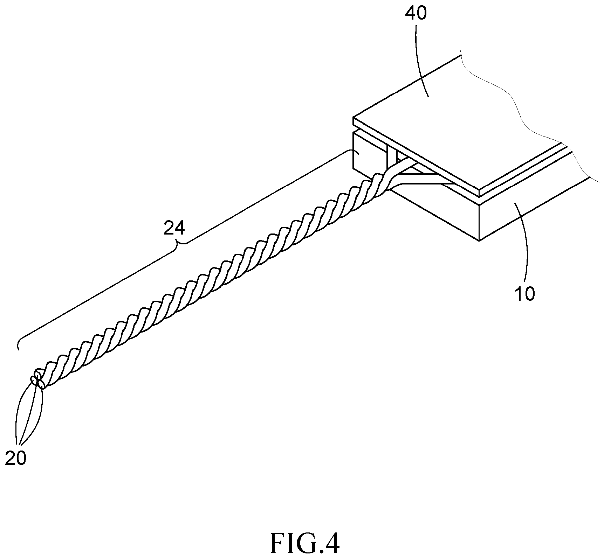

Please refer to FIG. 1 to FIG. 4 for the first embodiment of the present invention. The electrochemical sensor of the invention can be used to perform invasive detection on a host, and the host may be a human, an animal, or a plant. The electrochemical sensor can detect whether the host's body contains a target analyte, the concentration of the target analyte, and/or other to-be-detected values. The target analyte may be, for example but not limited to, glycated hemoglobin, blood sugar, a heavy metal, a nitrate, a nitrite, an allergen, formaldehyde, dissolved oxygen, uric acid, dopamine, ascorbic acid, potassium ferricyanide, acetaminophen, a halogen ion, a sulfur ion, an aqueous hydrogen peroxide solution, an arsenic(III) ion, a lead ion, a zinc ion, a chromium ion, phenols, an amino acid, or another similar compound. The to-be-detected value may be a physical parameter such as, but not limited to, a pH value or conductivity. The electrochemical sensor of the invention may also be implemented in such a way that it can be used in a non-invasive detection environment, e.g., to test a water sample taken from the environment or other aqueous solutions. In this embodiment, the electrochemical sensor includes a substrate 10, three wire electrodes 20, three gold fingers 30, and a cover plate 40.

The material of the substrate 10 may be, but is not limited to, polypropylene, polyethylene terephthalate, polyimide, polyethylene, polyurethane, or polycarbonate.

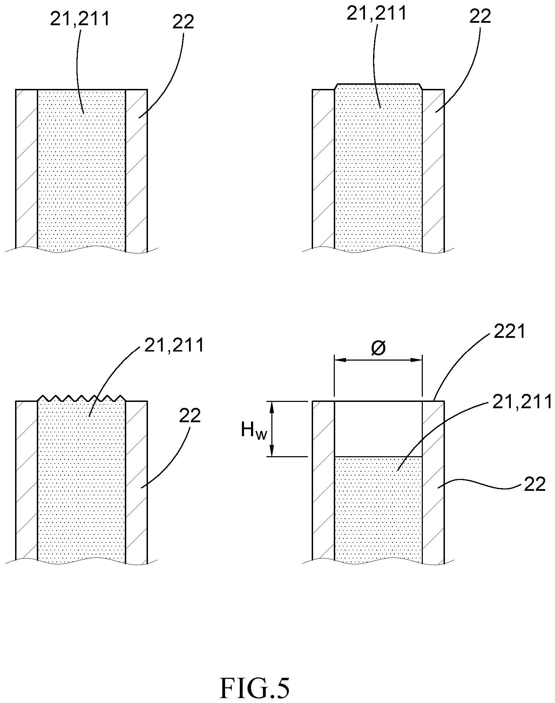

Each wire electrode 20 includes an electrically conductive core 21 and an insulating sheath 22 (see FIG. 5). Each insulating sheath 22 substantially covers the corresponding electrically conductive core 21 while exposing a proximal end 211 and a distal end 212, or free end, of the corresponding electrically conductive core 21. In addition, each wire electrode 20 has a substrate section 23 and an invasion section 24. The proximal end 211 and the distal end 212 of the electrically conductive core 21 of each wire electrode 20 are located at the substrate section 23 and the invasion section 24 of the wire electrode 20 respectively. The substrate section 23 of each wire electrode 20 is provided on the substrate 10. The invasion section 24 of each wire electrode 20 extends outward from an edge of the substrate 10, and the length for which each invasion section 24 extends outward from the edge may be greater than 10 mm. In this embodiment, the invasion sections 24 of the three wire electrodes 20 are wound around one another in a spiral manner. Winding the invasion sections 24 together in a spiral manner is advantageous in that the accuracy of detection is enhanced by the short distance and low electrical resistance among the electrodes. The electrically conductive core 21 in the invasion section 24 of at least one of the wire electrodes 20 is different in material from the electrically conductive cores 21 of the other wire electrodes 20. In this embodiment, for example, the electrically conductive cores of the three wire electrodes serve separately as a working electrode, an auxiliary electrode, and a pseudo electrode/reference electrode. Depending on the analyte to be detected, the electrically conductive cores of the three wire electrodes may by, but are not limited to, the materials listed in Table 1. The insulating sheaths 22, on the other hand, are made of an insulating material to prevent the electrically conductive cores of different wire electrodes from direct electrical connection with one another and hence from forming a short circuit.

**Table 1**

| Working electrode | Auxiliary electrode | Pseudo electrode/reference electrode | Analyte |
|---|---|---|---|
| Carbon | Carbon | Silver | Uric acid, dopamine, ascorbic acid, potassium ferricyanide, |
| Carbon | Platinum | Silver | Uric acid, dopamine, ascorbic acid, potassium ferricyanide, acetaminophen |
| Silver | Carbon | Silver chloride | Halogen ions |
| Nickle | Platinum | Carbon | Sulfur ion |
| Platinum | Platinum | Silver chloride | Aqueous hydrogen peroxide solution |
| Platinum | Gold | Silver chloride | Dissolved oxygen |
| Gold | Gold | Silver | Arsenic(III) ion, lead ion, zinc ion, chromium ion |
| Copper | Carbon | Silver | Phenols, amino acids |
| Bismuth | Carbon | Silver | Arsenic(III) ion, lead ion, zinc ion, chromium ion |

The wire electrodes can be viewed as metallic wire ultramicroelectrodes (MWUME) when the diameter ∅ of each electrically conductive core 21 is less than or equal to 25 µm, as metallic wire microelectrodes (MWME) when the diameter ∅ of each electrically conductive core 21 is greater than 25 µm and less than 1000 µm, or as metallic wire electrodes (MWE) when the diameter ∅ of each electrically conductive core 21 is greater than or equal to 1000 µm.

The gold fingers 30 are provided on the substrate 10. The electrically conductive core 21 in the substrate section 23 of each wire electrode 20 (e.g., the proximal end 211 of each electrically conductive core 21) is electrically connected to the corresponding gold finger 30. The electrical connection between each electrically conductive core 21 and the corresponding gold finger 30 may be formed by, for example but not limited to, soldering or application of electrically conductive tape. The gold fingers 30 may be printed on the substrate 10 by screen printing for example, and the material of the gold fingers 30 may be printed carbon paste or printed silver paste for example. The printed carbon paste or printed silver paste may further receive a surface treatment by, for example, being sputter-coated with a metal such as platinum, gold, copper, or silver. In this embodiment, the gold fingers 30 extend to an edge of the substrate 10.

The cover plate 40 is provided on the substrate 10 such that the entire substrate sections 23 of the wire electrodes 20 are fixed between the cover plate 40 and the substrate 10.

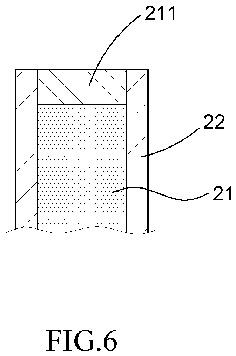

Referring to FIG. 5, the distal end of each electrically conductive core 21 may receive a surface treatment so as to have the configuration of being flush with the corresponding end face of the corresponding insulating sheath 22, or protruding from the corresponding end face of the corresponding insulating sheath 22, or having an irregular surface, or being sunken into the corresponding end face of the corresponding insulating sheath 22. One way of implementing the wire electrodes is to make the distal end 212 of each electrically conductive core 21 sunken into an end face 221 of the corresponding insulating sheath 22 while satisfying the relationship H_{w}/∅ < 50, where H_{w} is the depth to which the distal end 212 is sunken into the end face 221. This sunken configuration leaves room for subsequent chemical modification of the distal ends of the electrically conductive cores. For example, the recess formed at the aforesaid end face of each insulating sheath may be filled with an enzyme layer (not shown). Besides, referring to FIG. 6, the distal end 212 of at least one of the electrically conductive cores 21 is made of a material different from the material of the other portion of the electrically conductive core 21. For example, the distal end of this at least one electrically conductive core is made of carbon while the other portion of the electrically conductive core is made of copper in order to adapt to different detection environments.

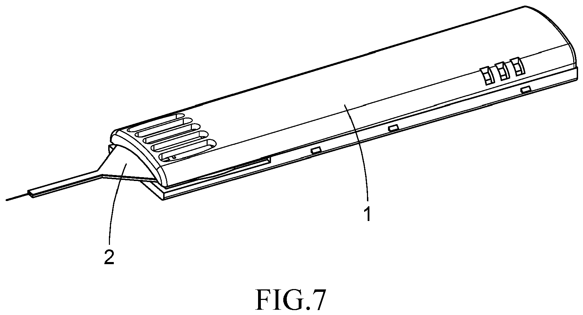

Referring to FIG. 7, the electrochemical sensor according to the embodiment shown in FIG. 1 to FIG. 4 can be used in combination with an electrochemical sensing relay 1. The electrochemical sensing relay 1 is configured to be electrically connected to each and every gold finger of the electrochemical sensor 2 and to relay the signals sensed by the electrochemical sensor to a remote receiving unit (e.g., a smartphone, a computer, or a cloud server) in order to perform further computation and/or display the computation result.

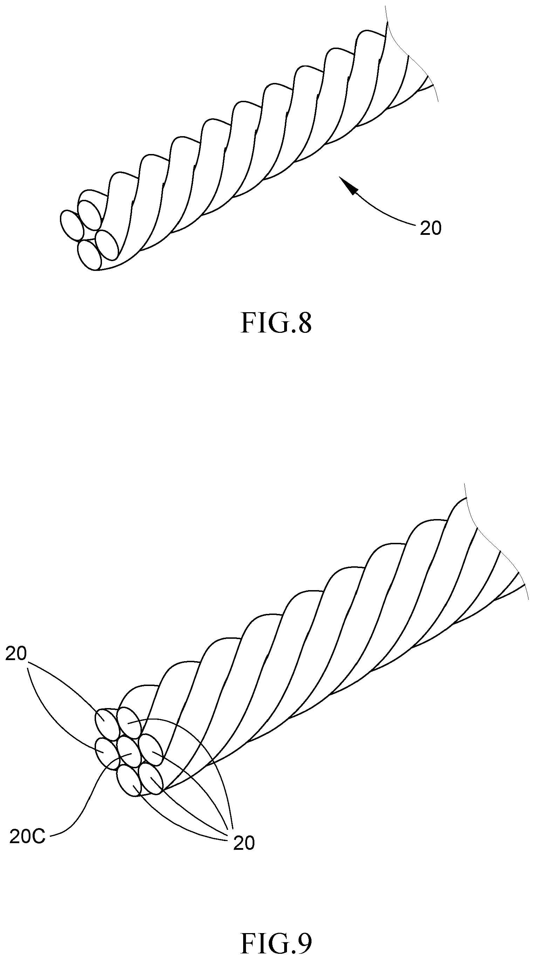

It should be pointed out that the number of the wire electrodes can be adjusted. For example, in the embodiment shown in FIG. 8, the spirally wound invasion sections of the four wire electrodes 20 can be used to detect more analytes at the same time than when fewer wire electrodes are provided, and as shown in FIG. 9, the invasion sections of six wire electrodes may be spirally wound around the invasion section of a central wire electrode 20c that extends along a straight line, i.e., with the invasion section of at least one wire electrode that extends along a straight line serving as an axis around which the invasion sections of the other wire electrodes are spirally wound.

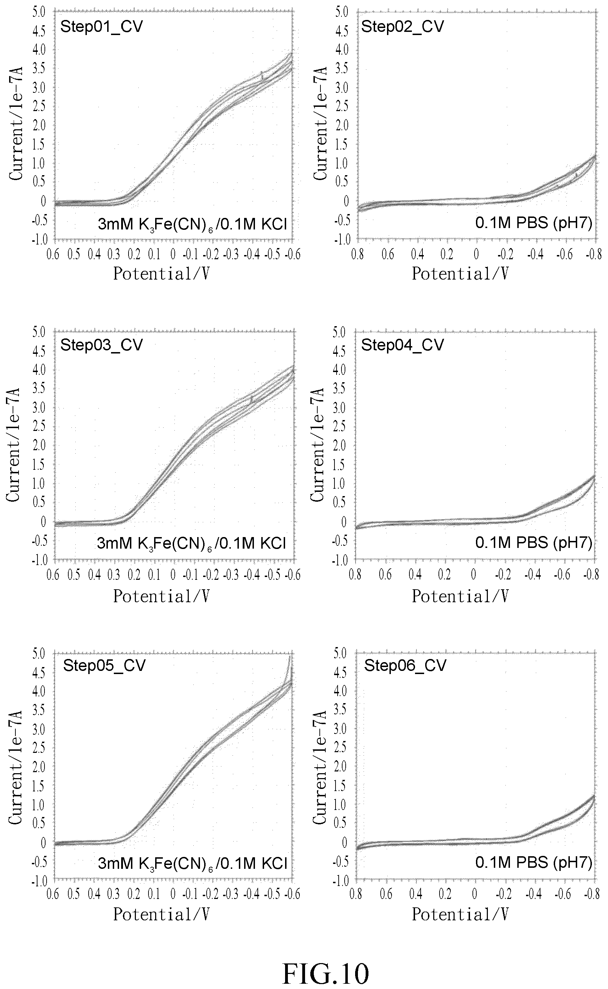

Referring to FIG. 10, a reproducibility test was conducted in which a plurality of electrochemical sensors as shown in FIG. 1 took turns being immersed in each of two different aqueous solutions for three times. The test results show that the detection result of each aqueous solution was highly reproducible with the electrochemical sensor of the present invention.

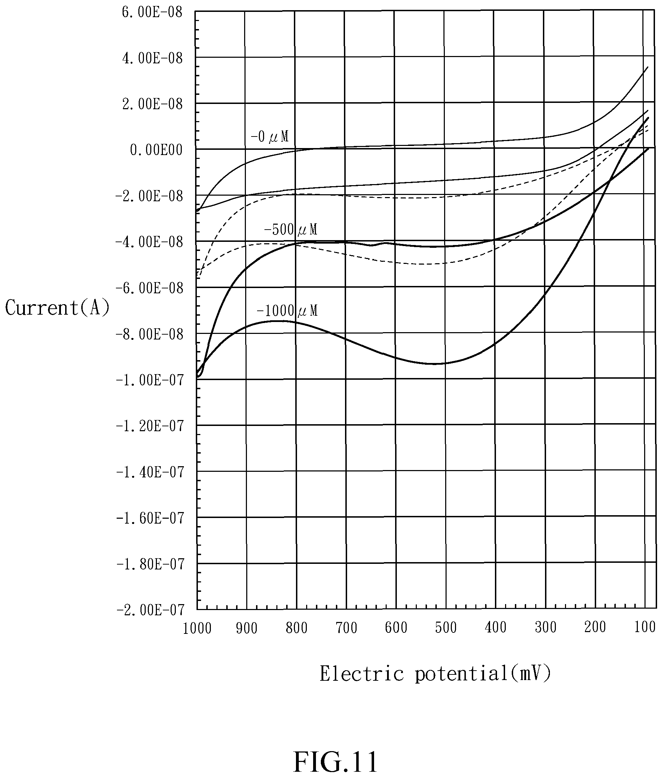

Referring to FIG. 11, an electrochemical sensor with three wire electrodes was immersed in 0.1 M PBS (phosphate-buffered saline), 500 µM aqueous hydrogen peroxide (H₂O₂) solution, and 1000µM aqueous hydrogen peroxide solution separately. The material of the major portions of the electrically conductive cores of the wire electrodes was carbon while the material of the distal ends of the electrically conductive cores was platinum. The test results show that the electrochemical sensor of the present invention was indeed capable of detecting different oxidation and reduction potentials in aqueous hydrogen peroxide solutions of different concentrations.

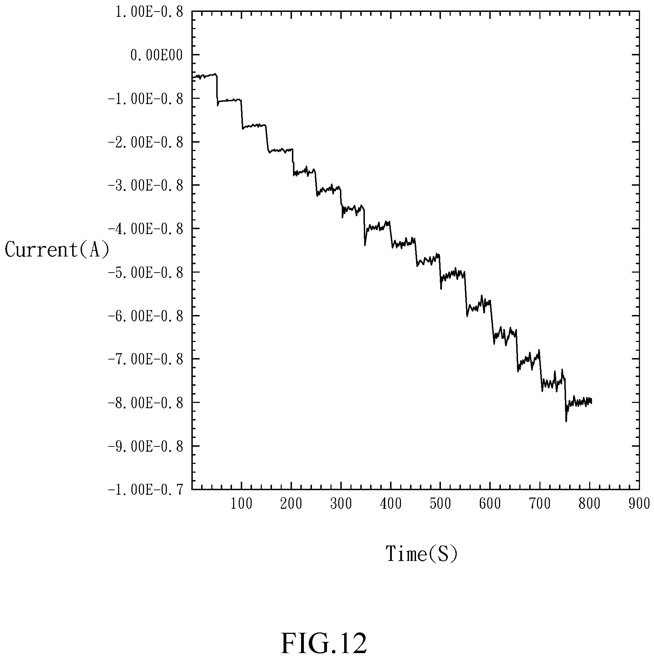

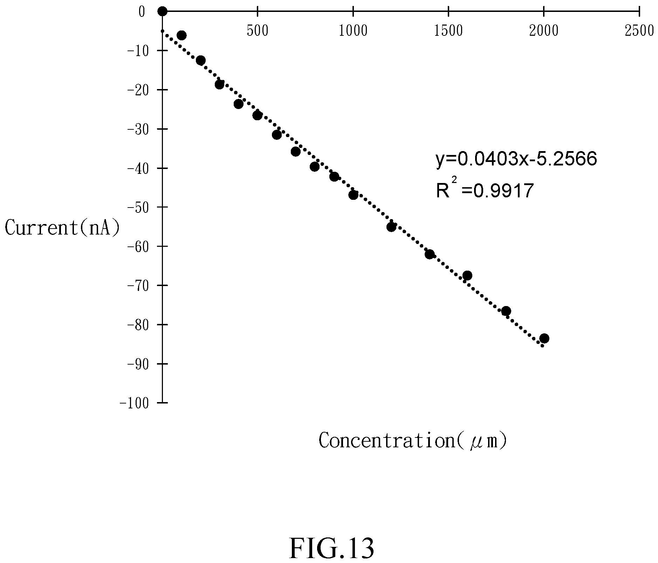

Referring to FIG. 12, the applicant used amperometry to test the performance of the electrochemical sensor of the present invention in detecting aqueous hydrogen peroxide solutions. The electrochemical sensor used in the test had three wire electrodes, with the material of the major portions of the electrically conductive cores of the wire electrodes being carbon, and the material of the distal ends of the electrically conductive cores being platinum. The applicant used amperometry together with the working parameters in Table 2 to detect aqueous hydrogen peroxide solutions of different concentrations. More specifically, the concentration of an aqueous hydrogen peroxide solution was adjusted every 50 seconds. Each of the first ten adjustments involved increasing the concentration by 100 µM, and each of the last five adjustments involved increasing the concentration by 200 µM. The final concentration was 2000 µM. The oxidation voltage was fixed at 600 mV. The test results show that the electrochemical sensor of the invention was able to detect the changes in concentration of the aqueous hydrogen peroxide solution sensitively, and that the detected current values are highly linearly correlated to the changes in concentration (see FIG. 13), indicating that the electrochemical sensor of the invention has high accuracy.

**Table 2**

| Elapsed time (s) | Concentration of aqueous hydrogen peroxide solution (µM) |
|---|---|
| 0 | 0 |
| 50 | 100 |
| 100 | 200 |
| 150 | 300 |
| 200 | 400 |
| 250 | 500 |
| 300 | 600 |
| 350 | 700 |
| 400 | 800 |
| 450 | 900 |
| 500 | 1000 |
| 550 | 1200 |
| 600 | 1400 |
| 650 | 1600 |
| 700 | 1800 |
| 750 | 2000 |

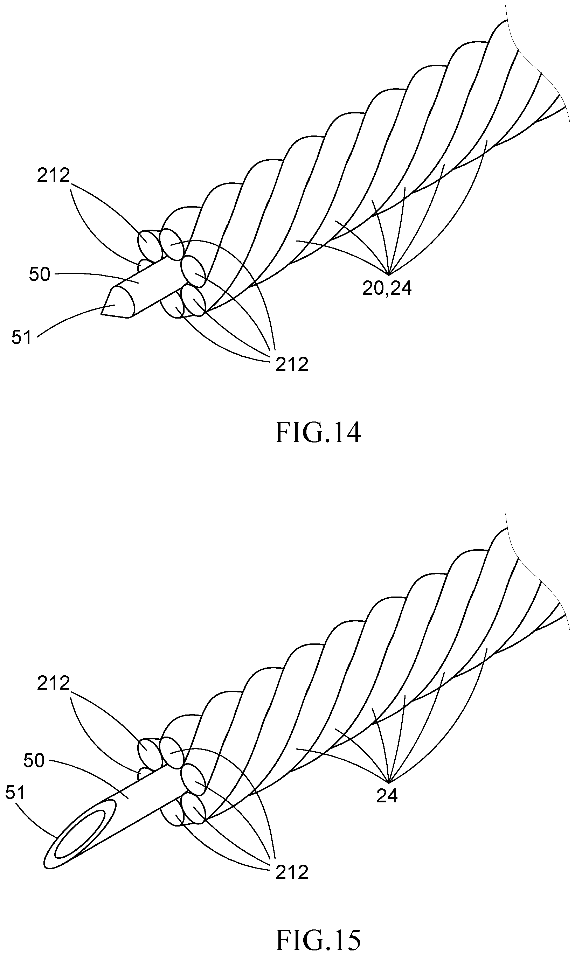

The embodiment shown in FIG. 14 is similar to the embodiment in FIG. 9, the major difference being that the electrochemical sensor in FIG. 14 includes an axle 50, and that the central wire electrode 20c in FIG. 9 is replaced by the axle 50 while the invasion sections 24 of the other wire electrodes 20 are wound around the axle 50 spirally. A portion of the axle is configured to be fixed to the substrate (not shown). The portion of the axle 50 around which the invasion sections 24 are spirally wound extends along a straight line. The axle 50 has a terminal end 51, and the terminal end 51 protrudes beyond the distal ends 212 of the invasion sections 24. In this embodiment, the terminal end 51 is solid and pointed and serves to change the structural strength of the invasion sections 24 so that the invasion sections 24 can be easily inserted into a detection target (e.g., soil or a plant) without the help of an additional placing device. The embodiment shown in FIG. 15 is similar to the embodiment in FIG. 14, the major difference being that the terminal end 51 of the axle 50 in FIG. 15 is hollow and pointed and serves also to change the structural strength of the invasion sections 24. The hollow terminal end 51 helps disturb the tissue into which it is inserted, so that the tissue can contact the distal ends 212 of the invasion sections 24 easily through capillary action for example, thereby facilitating detection. The axle 50 may be a non-metallic, non-conductor wire, whose material may be, for example, ceramic, silicon dioxide, plastic, acrylic, or a polymer such as polypropylene (PP), polycarbonate (PC), or polyethylene (PE).

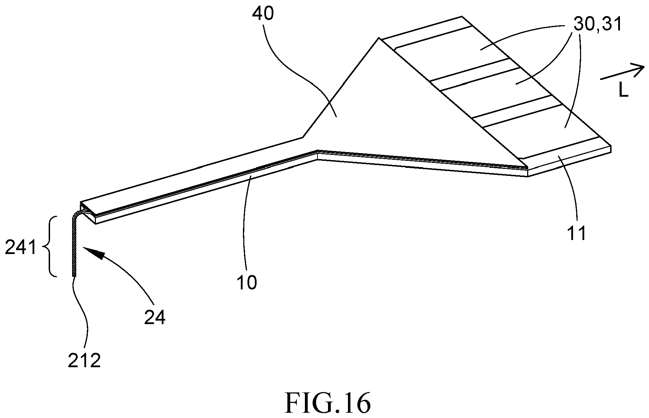

Referring to FIG. 16, the embodiment shown therein is similar to the embodiment in FIG. 1. The portion of each gold finger 30 that is not covered by the cover plate 40 is defined as an exposed section 31. The exposed sections 31 are parallel to one another and extend in an insertion direction L. The gold fingers 30 are provided on a working surface 11 of the substrate 10. The invasion section 24 of each wire electrode has a front-side portion 241, and the distal end 212 of the electrically conductive core of each wire electrode is located at the front-side portion of the invasion section of the wire electrode. The front-side portions 241 extend in a direction that is not parallel to the insertion direction L or the working surface 11. In this embodiment, the front-side portions 241 are perpendicular to the working surface 11. Another possible way of implementing the electrochemical sensor is to provide the invasion sections 24 with flexibility so that a user can adjust the extending direction of the front-side portions 241 according to the geometric properties of the detection target.

## Claims

1. An invasive multi-electrode electrochemical sensor, comprising:
a substrate;
a plurality of wire electrodes, wherein each said wire electrode comprises an electrically conductive core and an insulating sheath, each said insulating sheath substantially covers a corresponding one of the electrically conductive cores while exposing a proximal end and a distal end of the corresponding one of the electrically conductive cores, each said wire electrode has a substrate section and an invasion section, the proximal end and the distal end of the electrically conductive core of each said wire electrode are located at the substrate section and the invasion section of the each said wire electrode respectively, the substrate section of each said wire electrode is provided on the substrate, the invasion section of each said wire electrode extends outward from an edge of the substrate, and the invasion sections of the wire electrodes are at least partially wound around one another in a spiral manner; and
a plurality of gold fingers provided on the substrate, wherein the electrically conductive core in the substrate section of each said wire electrode is electrically connected to a corresponding one of the gold fingers, at least a part of the gold fingers is printed on the substrat by screen printing.

2. The invasive multi-electrode electrochemical sensor of claim 1, wherein the proximal ends of the wire electrodes are electrically connected to the gold fingers, respectively.

3. The invasive multi-electrode electrochemical sensor of claim 2, wherein the proximal ends of the wire electrodes are welded to the gold fingers, respectively.

4. The invasive multi-electrode electrochemical sensor of claim 1, wherein a diameter ∅ of each of the electrically conductive cores is less than or equal to 25 µm.

5. The invasive multi-electrode electrochemical sensor of claim 1, wherein a diameter ∅ of each of the electrically conductive cores is greater than 25 µm and less than 1000 µm.

6. The invasive multi-electrode electrochemical sensor of claim 1, wherein a diameter ∅ of each of the electrically conductive cores is greater than or equal to 1000 µm.

7. The invasive multi-electrode electrochemical sensor of claim 1, further comprising a cover plate provided on the substrate such that the substrate sections of the wire electrodes are at least partially fixed between the cover plate and the substrate.
